# EUROPEAN PATENT APPLICATION

(11) **EP 3 815 722 A1**
(43) Date of publication of application: **05.05.2021**
(21) Application number: 19841021.9
(22) Date of filing: 26.07.2019
(51) Int. Cl.: A61M 1/00

(54) **DEVICE FOR PROCESSING FATTY TISSUE AND USES THEREOF**

(30) Priority: 26.07.2018 ES 201830769
(71) Applicant: Disentropic S.L., 50004 Zaragoza (ES)
(72) Inventor: COHEN, David, 28015 Madrid (ES)
(74) Representative: Elzaburu S.L.P.
(86) International application number: PCT/ES2019/070529
(87) International publication number: WO 2020/021151

(57) **Abstract**

The present invention relates to a device for use in processing fatty tissue, characterised in that it comprises, in a closed circuit, a first tissue collection unit (1) connected to a second tissue collection unit (2), wherein the first tissue collection unit (1) has an opening (3) that allows the inlet of tissue and an opening (4) in the base of the first unit that allows the outlet of tissue, and wherein the second tissue collection unit (2) has an opening (6) in the base of the second unit that allows the outlet of tissue.

## Description

### BACKGROUND

Adipose or fatty tissue is a lax connective tissue mainly consisting of adipocytes, or fatty cells. Fatty tissue can be extracted from the body by liposuction and is useful for both aesthetic and reconstructive procedures, such as for isolating and treating the stromal-vascular fraction (SVF) or other fractions derived from centrifuging thereof.

Fatty tissue is extracted from the body by liposuction, where a hollow cannula comprising an opening at or near its free end is inserted in the area of the body to treat through a small incision in the skin. Fat cells are suctioned through the lumen of the cannula, which is connected to a vacuum source, and thereby led to a container. Once the fatty tissue has been obtained it can be used directly or manipulated to select the size of the fat globules, to isolate a specific group of cells, etc.

There is a need for simple, inexpensive devices that can be used for collecting the fatty tissue obtained in liposuction and allow them to be selected in a closed circuit.

### DESCRIPTION OF THE INVENTION

The present invention provides a simple and inexpensive solution, consisting in a device that, in a closed circuit, allows collecting and, if desired, selecting the fatty tissue obtained in liposuction according to the size of the fat globules. In addition, the device of the present invention allows separating the tissue from the liquid medium comprising the blood and anaesthetic, and even to wash said fatty tissue, at all times in a closed-circuit system.

Thus, in a first aspect the present invention relates to a device for use in processing fatty tissue characterised in that it comprises in a closed circuit a first tissue-collecting unit (1), connected to a second tissue-collecting unit (2), where the first tissue-collecting unit (1) has an opening (3) that allows the inlet of tissue and an opening (4) at the base of the first unit that allows the outlet of tissue, and where the second tissue-collecting unit (2) has an opening (6) at the base of the second unit that allows the outlet of tissue.

The fact that the device is a closed-circuit system has the advantage that the tissue being processed is not exposed to contamination by any external agents.

The openings of the device of the present invention allow the coupling of an extension or cannula due to their tubular shape.

In a preferred embodiment, the opening (3) is located at the upper part (8) of the first tissue-collecting unit (1).

In a preferred embodiment of the first aspect of the present invention, the first tissue-collecting unit (1) is connected to a second tissue-collecting unit (2) through a filter (7). Preferably, the filter (7) has a pore size of between 100 and 200 microns, preferably 150 ± 5 microns.

Preferably, the device (except for the filter) is made from a biocompatible plastic material, such as a thermoplastic polymer like polyethylene, polypropylene or a copolymer of polypropylene and ethylene, preferably a random copolymer of polypropylene and ethylene. The filter instead consists of a mesh preferably made from a biocompatible material, such as stainless steel, with 0.1 mm wire and an opening between 0.1 and 0.2 mm, preferably an opening of 0.15 ± 0.005 mm.

In another preferred embodiment of the first aspect of the present invention, the second tissue-collecting unit (2) presents at its upper part, immediately under the filter, an opening (5). Said opening (5) allows suction with a syringe or vacuum pump. Therefore, in another preferred embodiment of the first aspect of the present invention, the device also comprises means for creating a vacuum coupled to the opening (5).

In another preferred embodiment of the first aspect of the present invention, the device also comprises a cannula coupled to the opening (3). Preferably, the device also comprises a three-way valve coupled to the cannula. Said cannula attached to the three-way valve allows the direct transfer of the fatty tissue suctioned with a syringe to the first tissue-collecting unit (1).

In a second aspect, the present invention relates to the use of the device of the first aspect for processing fatty tissue. Preferably, said fatty tissue is used in biolipoplasty, obtaining the stromal-vascular fraction, isolating stem cells or isolating fractions of fine fatty tissue for treating fine wrinkles, rhinomodelling or manufacturing autologous cosmetics.

### DESCRIPTION OF THE FIGURES

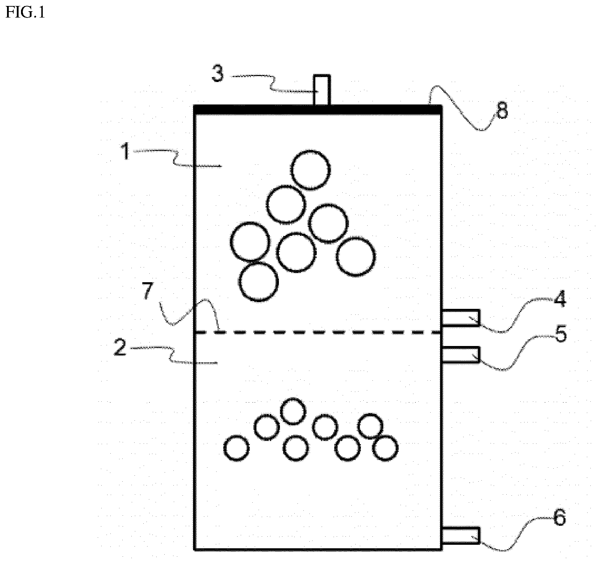

Figure 1. Device of the present invention.

### EXAMPLES

The invention will be better understood with reference to the following examples, although persons skilled in the art will easily appreciate that the specific examples described merely illustrate the invention.

### Example 1:

Connecting an extension of the usual infusion systems at one of its ends to the opening (3) and at its other end to a 3-way valve, and the latter in turn to a 20 or 50 cm³ syringe, the fatty tissue is suctioned and with the valve the content is transferred from the suction syringe to the first tissue-collecting unit (1). This is repeated as many times as needed to obtain the desired volume of tissue.

On one hand, if the volume of suctioned tissue is decanted under gravity onto the filter (7), at the first tissue-collecting unit (1) a complete fatty tissue is obtained with globules of different sizes, perfectly usable for lipotransfer, biolipoplasty, obtaining the stromal vascular fraction, isolation of stem cells, or isolation of other fractions of the fatty tissue.

It is also possible to obtain fatty tissue globules of different sizes with various aims if after suctioning with a syringe a vacuum is applied in the opening (5) to create a negative pressure inside the second tissue-collecting unit (2), which makes the finer fatty tissue globules pass through the filter.

After the fine fatty tissue supernatant is observed in the second tissue-collecting unit (2), the liquid fraction composed of a mixture of anaesthetic, blood, etc. is removed through the opening (6), as the fatty tissue floats on the liquid, which will exit first. In this way, after extracting the liquid fraction, it is possible to extract through the same opening (6) the fine fatty tissue and collect it directly in 1 or 3 cm³ syringes for direct use in the treatment of finer wrinkles, rhinomodelling in combination with plasma gel, manufacture of autologous cosmetics, etc.

On another hand, the device of the present invention can be used by suctioning or applying a vacuum at the opening (5) such that the thick fatty tissue is retained in the first tissue-collecting unit (1) and the fine fatty tissue remains in the second tissue-collecting unit (2).

Moreover, the device of the present invention allows washing the tissue, for example with serum, when it is inserted through the opening (3).

The fatty tissue of the first tissue-collecting unit (1) can be collected, for example, with a syringe through the opening (4), to be used in grafts, for example, or collected in a closed circuit in Falcon-type tubes connected to said opening for subsequent processing. For example, the fat obtained in this manner can be processed for obtaining stem cells by washing, centrifuging, etc.

## Claims

1. A device for use in processing fatty tissue, **characterised in that** it comprises in a closed circuit a first tissue-collecting unit (1), connected to a second tissue-collecting unit (2), where the first tissue-collecting unit (1) has an opening (3) on its upper part (8) and an opening (4) at its base, and where the second tissue-collecting unit (2) has an opening (6) at its base.

2. The device according to the preceding claim, where the first tissue-collecting unit (1) is connected to a second tissue-collecting unit (2) through a filter (7).

3. The device according to any of the preceding claims, where the first tissue-collecting unit (1) is connected to a second tissue-collecting unit (2) through a filter (7) that has a pore size of between 100 and 200 microns.

4. The device according to any of the preceding claims, where the second tissue-collecting unit (2) has an opening (5) at its upper part.

5. The device according to the preceding claim, additionally comprising means for creating a vacuum connected to the opening (5).

6. The device according to any of the preceding claims, additionally comprising a cannula connected to the opening (3).

7. The device according to the preceding claim, additionally comprising a three-way valve connected to the cannula.

8. Use of the device according to any of the preceding claims for processing fatty tissue.

9. Use according to the preceding claim, where the processing of fatty tissue is for use in biolipoplasty, obtaining the stromal-vascular fraction, isolating stem cells, or isolating fractions of fine fatty tissue for treating fine wrinkles, rhinomodelling or manufacturing autologous cosmetics.
